# EUROPEAN PATENT SPECIFICATION

(11) **EP 4 057 841 B1**
(45) Date of publication and mention of the grant of the patent: **04.09.2024**
(21) Application number: 20800682.5
(22) Date of filing: 09.11.2020
(51) Int. Cl.: A23C 21/00, A23L 33/19, A23L 33/00, A23J 1/20

(54) **USE OF ALPHA-LACTALBUMIN ENRICHED WHEY PROTEIN EXTRACT AS A SOURCE OF CHOLESTEROL AND SYNTHETIC NUTRITIONAL COMPOSITION COMPRISING AN ALPHA-LACTALBUMIN ENRICHED WHEY PROTEIN EXTRACT**
VERWENDUNG VON MIT ALPHA-LACTALBUMIN ANGEREICHERTEM MOLKENPROTEINEXTRAKT ALS EINE QUELLE VON CHOLESTERIN UND SYNTHETISCHE ERNÄHRUNGSZUSAMMENSETZUNG ENTHALTEND EINES MIT ALPHA-LACTALBUMIN ANGEREICHERTEM MOLKENPROTEINEXTRAKTS
UTILISATION D'EXTRAIT DE PROTÉINE DE LACTOSÉRUM ENRICHI EN ALPHA-LACTALBUMINE EN TANT QUE SOURCE DE CHOLESTÉROL ET COMPOSITION NUTRITIONNELLE SYNTHÉTIQUE COMPRENANT UN EXTRAIT DE PROTÉINE DE LACTOSÉRUM ENRICHI EN ALPHA-LACTALBUMINE

(30) Priority: 13.11.2019 EP 19208776
(43) Date of publication of application: 21.09.2022
(73) Proprietor: Société des Produits Nestlé S.A., 1800 Vevey (CH)
(72) Inventor: O'REGAN, Jonathan, Killarney, Co.Kerry V93E1T2 (IE); MOLONEY, Cian Terence, Cork City, Cork T12 XA4T (IE); O'CONNOR, Deborah, Limerick V94 EE0K (IE)
(74) Representative: Gagliardi, Tatiana
(86) International application number: PCT/EP2020/081445
(87) International publication number: WO 2021/094241

(56) References cited:
- WO-A1-2019/122363
- US-A- 4 782 138
- US-B1- 6 312 755
- W. W. WONG ET AL: "Effect of dietary cholesterol on cholesterol synthesis in breast-fed and formula-fed infants", JOURNAL OF LIPID RESEARCH, vol. 34, no. 8, 1 August 1993 (1993-08-01), US, pages 1403 - 1411, XP055253903, ISSN: 0022-2275
- ANTONIO CILLA ET AL: "Phospholipids in Human Milk and Infant Formulas: Benefits and Needs for Correct Infant Nutrition", CRITICAL REVIEWS IN FOOD SCIENCE AND NUTRITION, vol. 56, no. 11, 15 June 2015 (2015-06-15), USA, pages 1880 - 1892, XP055684763, ISSN: 1040-8398, DOI: 10.1080/10408398.2013.803951
- CIAN MOLONEY ET AL: "Polar lipid, ganglioside and cholesterol contents of infant formulae and growing up milks produced with an alpha lactalbumin-enriched whey protein concentrate", INTERNATIONAL DAIRY JOURNAL, 1 April 2020 (2020-04-01), GB, pages 104716, XP055684757, ISSN: 0958-6946, DOI: 10.1016/j.idairyj.2020.104716

## Description

### Field of the Invention

The present invention relates to the use of α-lactalbumin enriched whey protein extract as a source of cholesterol, and to the use of said α-lactalbumin enriched whey protein extract to optimize/configure the cholesterol concentration in a synthetic nutritional composition for an infant or child such that the cholesterol content resembles the content in human breast milk and ranges from 90 to 150 mg/L of the composition.

### Background to the Invention

Breast-feeding is recommended for all infants. However, in some cases breast-feeding is insufficient or not possible for medical reasons. In these situations infant formula can be used as a substitute for breast milk. However, studies have shown that the composition of infant formula is not identical to that of breast milk, and that it may not always have identical effects on the body. In light of this, and in light of the fact that breast milk is considered the gold standard when it comes to infant nutrition, a goal of infant formula manufacturers is to further develop the compositions of their infant formulas and growing-up milks and to bring them closer to breast milk.

Human milk is the ideal source of nutrition for infants, and is broadly composed of water, carbohydrates, lipids, proteins, vitamins and minerals. The lipid fraction of human milk occurs in various forms and is responsible for a wide variety of functions. The majority of lipids are in the form of neutral triglycerides (98%) that typically provide greater than 50% of the caloric intake of the newborn infant. Cholesterol and polar lipids (PLs) - primarily phospholipids and sphingolipids - make up a relatively small proportion of the lipids (0.2-2.0%) and are found mostly as part of the milk fat globule membrane (MFGM) (Jensen, Lipids, vol.34, issue 12, p.1243-1271, 1999).

Cholesterol is important for neuron and nervous system development, vitamin D synthesis and functions as a precursor of some bile acids (Beggio, Cruz-Hernandez, Golay, Lee & Giuffrida, J. Sep. Sci., 41(8), p.1805-1811, 2018; Ohlsson, Food Nutr. Res., 54(10), 2010). Human milk typically contains -90-150 mg/L cholesterol and it has been suggested that breast-feeding may be associated with lower total blood cholesterol concentrations later in life compared with formula feeding (Beggio et al., J. Sep. Sci., 41(8), p.1805-1811, 2018; Wong, Hachey, Insull, Opekun & Klein, J. Lipid Res., vol. 34, p. 1403-1411, 1993). Bovine milk is richer in cholesterol (~260 mg/L) than human milk, but infant formula typically contains low levels of cholesterol (<5 mg/L) as it is usually formulated with agara, J. gric. Food Chem., 63(32), p. 7245-7251, 2015; Precht, Food/Nahrung, 45(1), p. 2-8, 2001). Therefore, formula-fed infants have a reduced dietary cholesterol intake and have lower plasma cholesterol levels than breastfed infants (Wong et al., J. Lipid Res., vol. 34, p. 1403-1411,1993).

WO 2019/122363 discloses a process for manufacturing an α-lactalbumin enriched whey protein product but doesn't disclose the α-lactalbumin enriched whey protein product as a source of cholesterol.

Wong & al (Journal of lipid research, vol.34, n°8, p.1403-1411, 1993) discloses a study of the fractional synthesis rate 5FSR) of cholesterol measured in 6 breast-fed and 12 formula-fed infants. Wong & al doesn't disclose the use of a specific α-lactalbumin enriched whey protein extract as a source of cholesterol.

The present work describes the development nutritional formulations using an α-lactalbumin-enriched fraction at specific levels to be enriched in cholesterol such that formulations at the time of administration can meet the cholesterol levels of human breast milk (90 to 150 mg/l).
Surprisingly, the inventors have now found that a whey protein extract that has been processed to enrich the α-lactalbumin content by use of a particular process detailed herein comprises cholesterol in a concentration far higher than that found in other whey protein extracts using other methods or skimmed milk powder.

This advantageously enables WPE be used as a source of cholesterol and to optimize/configure the concentration of cholesterol in a composition for an infant or child, while at the same time also providing a source of α-lactalbumin. In the absence of an ingredient having both an enriched content of cholesterol and of α-lactalbumin, it would be required to add two different ingredients in order to add these two components into an infant formula. As cholesterol and α-lactalbumin are typically provided in the form of ingredients comprising significant amounts of additional milk protein, such as whey, the protein content in the infant formula in which two separate ingredients have been added would have a significantly higher protein content compared to a formula in which the present α-lactalbumin enriched whey protein extract is used. Thus, the present α-lactalbumin enriched whey protein extract (WPE) is particularly useful in infant formula intended to have a low protein content.

### Summary of the invention

In a first aspect, the present invention relates to use of an α-lactalbumin enriched whey protein extract (WPE) as a source of cholesterol in a synthetic nutritional composition for an infant or child wherein, said α-lactalbumin enriched WPE is obtained by a process comprising:
a. acidifying a whey protein product to pH 4 or below for example a pH in the rage 3.3 to 3.8 such as pH 3.5,
b. forming a low calcium whey protein product by concentrating the proteins in the acidified whey protein until the calcium to protein ratio is less than about 0.001 and
c. precipitating α-lactalbumin from the low-calcium whey protein product, wherein said precipitating step includes the sub-steps of:
   I. diluting the low-calcium whey protein product,
   II. adjusting the pH of the diluted low-calcium whey protein product to between 4 and 5 to form a precipitate and soluble proteins, and
   III. Separating the precipitate proteins from the soluble proteins for example by ultrafiltration e.g. through a 5K-50K molecular weight cut off membrane, and/or by diafiltration,
wherein the WPE is added to said composition in an effective amount sufficient to ensure that said synthetic nutritional composition has a final concentration of cholesterol found in human breast milk.

In step a, the whey protein product may be acidified by the addition of an acid. The acid may be a food grade acid e.g. hydrochloric acid, phosphoric acid, citric acid, and/or sulfuric acid. If a whey protein powder is used as the whey protein product, this must be brought into solution prior to step a. In one embodiment the step b) is performed for example by ultrafiltration e.g. through a 10K-100K molecular weight cut off membrane, and/or diafiltration.

The advantage of the present invention is using WPE, we can effectively limit the amount of protein in the synthetic nutritional composition such said composition is closer to human breast milk. Addition of other known dairy sources of cholesterol leads an undesirable increase in protein and energy requirements in a synthetic nutritional composition.

In one embodiment, the synthetic nutritional composition, in addition to WPE comprising cholesterol further comprises a2-β-casein.

In one embodiment, the synthetic nutritional composition, in addition to WPE comprising cholesterol further comprises sn-2 palmitate.

In one embodiment, the synthetic nutritional composition, in addition to WPE comprising cholesterol further comprises lactoferrin.

In a second aspect, the present invention relates to synthetic nutritional composition for an infant or child comprising an α-lactalbumin enriched whey protein extract as above-described wherein the synthetic nutritional composition comprises the WPE in a concentration in a range of 0.8 to 8 g/L and wherein the cholesterol concentration in said composition is at least 90 mg/L.

### Detailed description

A "α-lactalbumin enriched (WPE)" is herein defined as a whey protein extract that has been processed to increase the amount of α-lactalbumin in the whey protein extract compared to the un-processed whey protein extract.

The WPE obtained by the specific process described herein has been found to be surprisingly rich in cholesterol apart from α-lactalbumin and may advantageously be used to optimise the cholesterol concentration in a synthetic nutritional composition for an infant or child. The WPE may be added to a synthetic nutritional composition in an effective amount, sufficient to ensure that the said composition has a final concentration of cholesterol in a range found in human breast milk.

The invention also provides a synthetic nutritional composition comprising WPE, obtained as detailed herein, in a concentration of 0.8 to 8 g/L. A composition may for example comprise 4 to 5 g/L of WPE and may be formulated for an infant of 6 to 12 months of age, or may be formulated for an child of 12 to 36 months of age. The cholesterol concentration in said compositions may be at least 90 mg/L of the total reconstituted infant formula. The invention is set out in the amended set of claims.

The synthetic nutritional composition for an infant or child may be a composition for consumption by infants either alone or in combination with human breast milk, and may be an infant formula or human breast milk fortifier.

The WPE obtained as described herein (Lac B), or a synthetic nutritional composition comprising it, may be used to provide an infant or child with an optimized amount of cholesterol.

The whey protein product may be a whey protein concentrate prepared in any conventional way from mammalian whey (sweet or acid whey) for example cow, goat, sheep, buffalo, water buffalo, yak, human, camel and/or lama. A whey protein concentrate may for example be obtained from skimmed and/or clarified bovine whey that has been concentrated and/or desalted by common means, e.g. by ultrafiltration (the whey protein product may be the retentate) and/or diafiltration.

A process for obtaining an α-lactalbumin enriched WPE for use in the invention detailed herein is described in US 6 312 755.

Accordingly in an embodiment of the present invention the WPE is rich in cholesterol and comprises at least 4 g, preferably at least 4.5g, more preferably at least 5 g, even more preferably at least 5.5 g and most preferably 5.7 g of cholesterol per 100 g. In a preferred aspect of the invention, the amount of cholesterol is determined in accordance with the method described in Example 1 below.

The term "cholesterol" as used herein preferably refers to a lipid structure consisting of four fused hydrocarbon rings, a hydrocarbon tail and a hydroxyl group, which is an important structural component of animal cell membranes.

In another aspect, the present invention relates to the use of an α-lactalbumin enriched WPE to optimise the cholesterol concentration of a synthetic nutritional composition for an infant or child wherein said α-lactalbumin enriched WPE is obtained by a process as detailed herein. The invention is set out in the appended set of claims.

The α-lactalbumin enriched WPE may be added to a synthetic nutritional composition in any amount effective (an effective amount) to optimise the concentration of cholesterol in said synthetic nutritional composition for an infant or child.

The term "A2 milk" generally refers to a variety of cow's milk that mostly lacks a form of β-casein proteins called A1, and instead has mostly the A2 form. A2 cow's milk is commercially available from the a2 Milk Company (Auckland, New Zealand). Non-cow milk, including that of humans, sheep, goats, donkeys, yaks, camels, buffalo, and others, also contain mostly A2 β-casein, and so the term "A2 milk" is also used in that context.

Given that human breast milk is the gold standard when it comes to infant and/or child nutrition, the concentration of cholesterol in a synthetic nutritional composition for an infant or child may be considered optimised if the concentration of cholesterol is within a range, or above a range, found in human breast milk. Cholesterol is found in animal products, such as meat and poultry, but these are not suitable for inclusion in infant formula. Accordingly, optimisation of the cholesterol level of a synthetic nutritional composition can be most practically achieved through the inclusion of cholesterol-rich dairy materials.

A particular advantage of the WPE used in the invention is that it can provide an optimised amount of cholesterol to a synthetic nutritional composition for an infant or child and negate the need to add additional ingredients for this purpose, for example additional ingredients whose addition would be for the sole or primary purpose of increasing the cholesterol concentration e.g. isolated cholesterol. Accordingly, in an embodiment the α-lactalbumin enriched WPE is not used in combination with an additional ingredient whose sole or primary purpose would be to increase the cholesterol concentration in the synthetic nutritional composition, for example it is not used in combination with isolated cholesterol as the levels achieved through the use of WPE are already within the target range.

The term "infant" as used herein refers to a human infant of up to 12 months of age and includes preterm and very preterm born infants, infants having a low birth weight i.e. a new born having a body weight below 2500g (5.5 pounds) either because of preterm birth or restricted fetal growth, and infants born small for gestational age (SGA) i.e. babies with birth weights below the 10th percentile for babies of the same gestational age.

The term "child" as used herein refers to a human of 1 to 18 years of age, for example a human of 1 to 8 years of age, a human of 1 to 3 years of age, and/or a human of 1 to 2 years of age.

A "preterm" or "premature" means an infant or young child that was not born at term. Generally it refers to an infant born prior to the completion of 37 weeks of gestation.

The WPE formed by the process described herein also serves as a source of protein for example α-lactalbumin, β-lactoglobulin, lactoferrin and immunoglobulins.

The WPE obtained by this process set out hereinabove comprises α-lactalbumin in a concentration in the range of about 28% to 40% of the total protein, for example about 28% to 36% of total protein, and β-lactoglobulin in a concentration in the range of about 8% to about 33% of the total protein, for example 10% to about 29% of total protein. The percentage of α-lactalbumin in the WPE is greater than the percentage of β-lactoglobulin, for example the β-lactoglobulin concentration is not greater than the percentage of the α-lactalbumin content minus 7%.

All percentages disclosed herein with respect to the α-lactalbumin enriched WPE are on a w/w basis unless stated otherwise.

The term "optimised concentration of α-lactalbumin" as used herein refers to a concentration of α-lactalbumin that is within a range found in human breast milk (1.6-3.8 g/L). The α-lactalbumin enriched WPE may be considered to optimise the concentration of α-lactalbumin in a synthetic nutritional composition if the α-lactalbumin concentration for the synthetic nutritional composition is within the range found in human breast milk, when considering other ingredients comprised in the composition that comprise α-lactalbumin e.g. dairy ingredients such as skimmed milk powder and whey protein. Said ingredients may comprise α-lactalbumin innately.

In an embodiment the synthetic nutritional composition comprises the WPE in a concentration within a range of 0.8 to 10 g/L for example 0.8 to 8, 0.8 to 5 g/L, 0.85 to 4.5g/L, 3 to 4.5g/L.

In an embodiment at least 10% of the total cholesterol in the synthetic nutritional composition comes from the WPE for example 10 to 100%, 49% to 100%, 49% to 70%.

The synthetic nutritional composition comprises cholesterol in a concentration in a range as in human breast milk or in a higher concentration. Accordingly, the synthetic nutritional composition may comprise cholesterol in a concentration of 10 mg/L or more, for example from 10 to 400 mg/L, or from 10 to 350 mg/L.

In another embodiment of the present invention the composition also comprises α-lactalbumin in a concentration within a range found in human breast milk for example in a range of 1.6 to 3.8 g/L, for example 1.7 to 3 g/L.

A goal of infant formula manufacturers is to mimic the composition of human breast milk. However, the composition of human breast milk is extremely dynamic and changes over time. For this reason synthetic nutritional compositions for infants or children are usually stage based with a particular stage being suitable for use in infants or children falling within a particular age range e.g. stage 1 may be aimed at infants of 0 to 6 months, stage 2 may be aimed at infants of 6 months to 12 months, stage 3 may be aimed at children of 12 to 36 months, stage 4 may be aimed at children of 3 to 8 years. Each stage is formulated so that its composition is considered nutritionally sound with respect to the age range of the infant or child to whom it is directed.

In an embodiment of the present invention there is provided a synthetic nutritional composition for an infant or child comprising 9 to 10 g/L of the α-lactalbumin enriched WPE used in the invention for example 9.5-10.2 g/L. In an embodiment said composition is formulated for an infant of 0 to 12 months. In a more specific embodiment the total concentration of cholesterol in said composition is at least 90 mg/L and more specifically in a range of 90 to 150 mg/L.

In a preferred embodiment, the synthetic nutritional composition has a low total protein content. The amount of protein can be as low as adequate for the type of composition and the individual intended to consume it, such as for example according to nutritional requirements and/or regulations. For example the protein content is of at most 20 g/L, preferably at most 18g/L, more preferably at most 16 g/L, even more preferably at most 15g/L, even more preferable 14g/L, even more preferably between 5 and 20 g/L, even more preferably 8 to 18 g/L, even more preferably 10 to 16g/L, more preferably 11 to 15g/L, more preferably 12 to 14 g/L, such as for example 13.5 g/L.

The synthetic nutritional composition for an infant or child can also comprise any other ingredients or excipients known to be employed in the type of synthetic nutritional composition in question e.g. infant formula.

Non limiting examples of such ingredients include: other proteins, amino acids, carbohydrates, oligosaccharides, lipids, prebiotics or probiotics, essential fatty acids, nucleotides, nucleosides, vitamins, minerals and other micronutrients.

Other suitable and desirable ingredients of synthetic nutritional compositions, that may be employed in the synthetic nutritional compositions for infants or children are described in guidelines issued by the Codex Alimentarius with respect to the type of synthetic nutritional composition in question e.g. Infant formula, growing up milk, HM fortifier, follow on formula, or food stuffs intended for consumption by infants e.g. complementary foods.

The WPE may be added to a synthetic nutritional composition for an infant or child by simply mixing it with other ingredients included in the composition.

Non limiting examples of synthetic nutritional compositions for an infant or child are infant formula, a growing up milk, a composition for infants that is intended to be added or diluted with human breast milk, or a food stuff intended for consumption by an infant and/or child either alone or in combination with human breast milk.

α-lactalbumin is rich in essential and conditionally essential amino acids. Accordingly, the α-lactalbumin enriched WPE of the invention is particularly suitable in low protein synthetic nutritional compositions for infants and children because for a minimal protein intake said infants and children still intake sufficient amino acids to optimise growth and development i.e. be within standard growth curves e.g. WHO standard growth curves.

The WPE used in the present invention and obtained by the process described herein contains a minimum amino acid concentration, in grams per 100 grams of total protein, as follows: arginine 3.1; cystine 1.4; histidine 1.6; isoleucine 1.0; leucine 5.3; lysine 3.9; methionine 0.3; phenylalanine 1.2; threonine 3.2; tryptophan 1.5; tyrosine 0.9; and valine 1.0. Accordingly, it may not be necessary to add amino acids to the synthetic nutritional compositions for infants or children when using this WPE. The non-protein nitrogen content may be about 15% or less of total nitrogen. The total protein content may be between about 12.5% to about 95% for example 35% to 80% or 73% to 77%. The fat content may be about 15% or less. The ash content may be about 4.5% or less.

In an embodiment the synthetic nutritional composition is a low protein infant formula. A low protein infant formula will comprise less than 3.5g of protein /100kcal for example less than 2.5g/100kcal or less than 2g/100kcal. The low protein infant formula may be an infant formula formulated for an infant of up to 12months of age, for example for an infant of 0 to 6 months of age, or an infant of 6 to 12 months of age.

The synthetic nutritional compositions for infants or children may be prepared by methods well known in the art for preparing the type of synthetic nutritional composition in question e.g. infant formulae, follow on formulae, a composition for infants that is intended to be added or diluted with HM e.g. HM fortifier, or food stuffs intended for consumption by infants either alone or in combination with HM e.g. complementary foods.

An infant formula may for example be prepared by blending appropriate quantities of the α-lactalbumin-enriched whey protein concentrate with skimmed milk, lactose, vegetable oils and fat soluble vitamins in water. These materials may be blended together in quantities sufficient to provide a final concentration of approximately 400 g/L Mineral salts may then be added to the mixture prior to a high temperature/short time pasteurization step. Appropriate mineral salts include calcium chloride, calcium carbonate, sodium citrate, potassium hydroxide, potassium bicarbonate, magnesium chloride, ferrous sulfate, potassium citrate, zinc sulfate, calcium hydroxide, copper sulfate, magnesium sulfate, potassium iodide, sodium selenite, etc. The mixture may then be homogenized and cooled. Heat-labile vitamins and micronutrients may then be added to the mixture. The mixture may then be standardized with deionized water to a final total solids concentration of about 120 to about 135 for example about 129 g/L, which is equivalent to about 670 kcal per litre. The formula may then be sterilized using a conventional ultrahigh temperature or standard retort process. This sterilized material may then be placed in appropriate packaging.

In another aspect of the present invention there is provided the use of the α-lactalbumin enriched whey protein extract obtained as disclosed herein to provide an optimised amount of cholesterol to an infant or child. As disclosed herein, said α-lactalbumin enriched whey protein extract may be added to a synthetic nutritional composition in an amount effective to provide an optimised concentration of cholesterol. The α-lactalbumin enriched whey protein extract obtained as disclosed herein may also provide an optimised amount of α-lactalbumin to an infant or child.

Because human breast milk is the gold standard when it comes to infant nutrition, and because the synthetic nutritional compositions comprising the α-lactalbumin enriched whey protein extract disclosed herein may comprise cholesterol in an optimized concentration, they may be used to provide an optimum amount of cholesterol to an infant and thereby to ensure optimum cholesterol levels in an infant or child.

Accordingly, in another aspect not according to the present invention there is provided a synthetic nutritional composition for an infant or child for use to treat or prevent sub-optimal growth and development, wherein said synthetic nutritional composition comprises WPE obtained as disclosed herein in an amount effective to provide an optimised concentration of cholesterol. The composition may be a composition described herein.

As disclosed hereinabove, the the synthetic nutritional compositions comprising WPE disclosed herein may also comprise α-lactalbumin in an optimized concentration. Consequently, they may also be used to provide an optimum amount of α-lactalbumin to an infant or child and thereby ensure optimum α-lactalbumin levels in an infant or child.

There now follows a series of non-limiting examples that serve to illustrate the invention.

### Example 1

### Raw materials & nutritional powder products

### Infant formula raw materials

Skimmed milk powder (SMP) samples were obtained from each of four suppliers: one located in Ireland (SMP A; *n* = 5), one in Germany (SMP B; *n* = 5), one in the USA (SMP C; *n* = 5) and one in New Zealand (SMP D; *n* = 5). WPC samples (35% protein) were obtained from each of two suppliers; one located in Ireland (WPC A; *n* = 5) and one in the USA (WPC B; *n* = 5). WPC ingredients enriched in α-lactalbumin was obtained from two sources (i) Lac A; *n* =2 and (ii) Lac B; *n* = 5. Lac A was produced using a membrane process to selectively retain components larger than α-lactalbumin, thereby enriching the permeate in the target protein. As part of the manufacturing process of Lac B, α-lactalbumin was enriched by acid precipitation at reduced temperature (~4 °C), followed by a membrane filtration step to enrich the retentate in α-lactalbumin, as well as other relatively large molecules, including fat globules.

### 2.1.2 Nutritional formula powders

Prototype infant formula (IF) or growing up milk (GUM) powders were produced in a pilot plant facility as follows: Dry ingredients (whey source of WPC A and/or Lac B, SMP, lactose, lipids and soybean lecithin) were added sequentially to reverse-osmosis pre-treated water (~70 °C) and mixed by agitation until dissolved. The resulting mixtures (-35% solids) were then homogenised (two-stage homogeniser: 2500 psi and 500 psi for the first and second stages, respectively) heat-treated using a plate heat-exchanger (85 °C x 22 sec holding time) and cooled to 10 °C. The mixture was then stored for 16 h at 10 °C, before the liquids were heated to ~50 °C and spray dried using a small-scale dryer to produce powders for analysis.

IF prototypes, suitable for infants from birth up to approximately 6 months age, were produced to contain as a whey protein source either Lac B (IF 1; *n* = 5), or WPC A (IF 2; *n* = 2). The whey protein source and SMP quantities were set to target the same protein content (10.4 g/100 g, 60:40 whey: casein ratio), and the lactose content was then varied as necessary to achieve the lactose target (53.0 g/100 g); the ingredients used were otherwise identical in both products and included anhydrous milk fat (45% of blend) and a combination of vegetable oils (55% of blend; as rapeseed, high oleic sunflower, regular sunflower and coconut oils) as lipid sources.

GUM prototypes, suitable for children of approximately one to three years of age, were produced to contain as a whey source either a mixture of Lac B and WPC A (GUM 1; *n* = 5) or only WPC A (GUM 2; *n* = 3). Both GUM products targeted a protein target of 13.7 g/100 g (40:60 whey:casein), and the lactose content was then varied as necessary to achieve the available carbohydrate target (61.0 g/100g); the ingredients used were otherwise identical in both products and included a combination of vegetable oils (soybean, high oleic sunflower and palm oils) as lipid sources.

### Cholesterol quantification

Cholesterol was quantified by AOAC Official Method 994.10 for measuring cholesterol in food products. Briefly, samples were saponified by refluxing with an ethanol/KOH solution, and the unsaponified fraction containing cholesterol was extracted with high-purity toluene. Cholesterol was derivatised to trimethylsilyl ethers, separated on a cross-linked 5% phenyl-methyl silicone capillary column (0.17 µm film thickness) and quantified by flame-ionisation-detector gas chromatography.

### Cholesterol content of ingredients

All of the SMP samples analysed had similar cholesterol levels, ranging from 20.7 to 24.2 mg/100 g (Table 1). Assuming liquid skimmed milk to contain 91% water and 9% solids this corresponds with a range of approximately 186-218 mg/L in the liquid product; this is lower than, but reasonably close to, reported levels of ~260 mg/L for whole bovine milk (Precht, 2001). This suggests that most of the milk cholesterol is retained with other Milk fat globule membrane (MFGM) type components in the skimmed milk phase, rather than being lost in the cream phase during fat skimming.

WPC A and B were higher in cholesterol than the SMP samples, containing mean levels of 77.0 and 85.3 mg/100 g, respectively; this was likely as a result of these materials having higher lipid concentrations than SMP. Although the basic concepts are consistent, not all WPC ingredients are produced necessarily with the same procedure; so as well as potential genetic and feeding differences to give different milk compositions, processing differences may be a further differentiating factor between the WPCs analysed. The mean cholesterol level of Lac B (556 mg/100 g) was the highest level of any of the ingredients analysed; this high level was likely as a result of enrichment of MFGM type components, as described previously for PLs. The low cholesterol level in Lac A (42.2mg/100g) corresponded with the low PL and gangliosides levels, and was likely due the low fat content of the material.

**Table 1. Mean and cholesterol contents of skimmed milk powder (SMP), whey protein concentrate (WPC) and α--lactalbumin enriched WPC (Lac) powders. Analysed values are presented as mean ± relative standard deviation of data; sample size (n) is indicated in parentheses.**

| Material | Cholesterol mg/100 g |
|---|---|
| SMP A (*n* = 5) | 24.2 ± 18.0% |
| SMP B (*n* = 5) | 23.1 ± 16.4% |
| SMP C (*n* = 5) | 20.7 ± 18.1% |
| SMP D (*n* = 5) | 21.9 ± 20.3% |
| WPC A (*n* = 5) | 77.0 ± 7.2% |
| WPC B (*n* = 5) | 85.3 ± 11.5% |
| Lac A (*n* = 2) | 42.2 |
| Lac B (*n* = 5) | 556 ± 5.1% |
| * | |

### 3.2.3 Cholesterol content of Infant formula (IF) and Growing-up-milk (GUM)

Cholesterol levels were higher in IF 1 and GUM 1 than IF 2 and GUM 2, respectively (Table 2), in line with the presence of Lac B. Despite the absence of Lac B in IF 2, it still contained a reasonably high level cholesterol; this was due to the presence of anhydrous milk fat in the formulation. GUM 2, which did not contain Lac B or anhydrous milk fat, had the lowest cholesterol content.

The cholesterol level in IF 1 (10 mg/100 mL) was similar to reported human milk levels; for example, Beggio et al. (2018) found mature human milk to have a mean cholesterol content of 11.3 ± 3.0 mg/100 g To the authors' knowledge, this study is the first infant formula with reported cholesterol levels similar those of human milk, with commercial formulations typically containing ~5 mg/100 mL (Claumarchirant al., 2015). The higher cholesterol intake of breastfed infants is associated with several benefits, including enhanced modulation of cholesterol metabolism in infancy, and reduced plasma low-density lipoprotein levels (and associated reduced risk of cardiovascular disease) in adulthood (Delplanque, Gibson, Koletzko, Lapillonne & Strandvik, 2015). Supplying similar amounts of cholesterol to formula fed infants may potentially result in similar benefits.

**Table 2. Mean levels of cholesterol in infant formula prototypes produced using either Lac B (IF 1) or Lac A (IF 2), and growing-up milk prototypes produced with a combination of WPC A and Lac B (GUM 1) only WPC A (GUM 2). Analysed values are presented as mean ± relative standard deviation of data; sample size (n) is indicated in parentheses. Nutrient levels per 100 mL are based on the reconstitution instructions for the product: 12.9 g/100 mL for IF and 16.9 g/100 mL for GUM.**

| Nutritional Powder Product Stage | Cholesterol | |
|---|---|---|
| | mg/100 g | mg/100 mL |
| IF 1 (*n* = 5) | 77.4 ± 2.86% | 10.0 ± 2.86% |
| IF 2 (*n* = 5) | 41.6 ± 4.85% | 5.37 ± 4.85% |
| GUM 1 (*n* = 5) | 20.8 ± 4.5% | 3.51 ± 4.5% |
| GUM 2 (*n* = 3) | 15.9 ± 3.6% | 2.68 ± 3.6% |

## Claims

1. Use of an α-lactalbumin enriched whey protein extract (WPE) as a source of cholesterol in a synthetic nutritional composition for an infant or child, wherein the α-lactalbumin enriched WPE is obtained by a process comprising:
a. acidifying a whey protein product to pH 4 or below
b. forming a low calcium whey protein product by concentrating the proteins in the acidified whey protein until the calcium to protein ratio is less than about 0.001 and,
c. precipitating α-lactalbumin from the low-calcium whey protein product, wherein said precipitating step includes the sub-steps of:
(I) diluting the low-calcium whey protein product,
(II) adjusting the pH of the diluted low-calcium whey protein product to between 4 and 5 to form a precipitate and soluble proteins, and
(III) Separating the precipitate proteins from the soluble proteins;
Wherein the WPE is added to said composition in an effective amount sufficient to ensure that said synthetic nutritional composition has a final concentration of cholesterol found in human breast milk.

2. Use according to claim 1 wherein the cholesterol content in synthetic nutritional composition ranges from 90-150 mg/L.

3. Use according to claims 1 or 2 wherein the WPE is also used to optimise the concentration of α-lactalbumin in said synthetic nutritional composition for an infant or child, and wherein said WPE is added to said composition in an effective amount sufficient to provide α-lactalbumin in one of the ranges found in human breast milk.

4. A synthetic nutritional composition for an infant or child comprising an α-lactalbumin enriched whey protein extract as defined in claim 1 wherein the synthetic nutritional composition comprises the WPE in a concentration in a range of 0.8 to 8 g/L and wherein the cholesterol concentration in said composition is at least 90 mg/L.

5. A synthetic nutritional composition for an infant or child according to claim 4 wherein said composition is an infant formula comprising 4 to 5 g/L of WPE and wherein said composition is preferably formulated for an infant of 6 to 12 months of age.

6. A synthetic nutritional composition for an infant or child according to claim 4 wherein said composition is an infant formula comprising 4 to 5 g/L of WPE and wherein said composition is preferably formulated for a child of 13 to 36 months of age.

7. A synthetic nutritional composition for an infant or child according to claim 4 wherein said composition is an infant formula comprising 0.5 to 1.5 g/L of WPE and wherein said composition is preferably formulated for a child of 3 to 8 years of age.

8. A synthetic nutritional composition for an infant or child according to any one of claims 4 to 7 wherein the synthetic nutritional composition for an infant or child is a composition for consumption by infants either alone or in combination with human breast milk and is preferably an infant formula or human breast milk fortifier.

## Patentansprüche

1. Verwendung eines a-Laktalbumin-angereicherten Molkenproteinextrakts (WPE) als eine Cholesterinquelle in einer synthetischen Nährstoffzusammensetzung für einen Säugling oder ein Kind, wobei der α-Laktalbumin-angereicherte WPE durch einen Prozess erhalten wird, umfassend:
a. Ansäuern eines Molkenproteinprodukts auf einen pH-Wert von 4 oder darunter
b. Ausbilden eines kalziumarmen Molkenproteinprodukts durch Konzentrieren der Proteine in dem angesäuerten Molkenprotein, bis das Verhältnis von Kalzium zu Protein weniger als etwa 0,001 beträgt und,
c. Ausfällen von α-Laktalbumin aus dem kalziumarmen Molkenproteinprodukt, wobei der Ausfällungsschritt die Teilschritte einschließt, zum:
(I) Verdünnen des kalziumarmen Molkenproteinprodukts,
(II) Anpassen des pH-Werts des verdünnten kalziumarmen Molkenproteinprodukts auf zwischen 4 und 5, um eine Ausfällung und lösliche Proteine auszubilden, und
(III) Trennen der Ausfällungsproteine aus den löslichen Proteinen;
wobei der WPE zu der Zusammensetzung in einer wirksamen Menge hinzugefügt wird, die ausreicht, um sicherzustellen, dass die synthetische Nährstoffzusammensetzung eine Endkonzentration von Cholesterin aufweist, die in menschlicher Muttermilch gefunden wird.

2. Verwendung nach Anspruch 1, wobei der Cholesteringehalt in der synthetischen Nährstoffzusammensetzung von 90 bis 150 mg/L liegt.

3. Verwendung nach den Ansprüchen 1 oder 2, wobei der WPE ebenso verwendet wird, um die Konzentration von α-Laktalbumin in der synthetischen Nährstoffzusammensetzung für einen Säugling oder ein Kind zu optimieren, und wobei der WPE zu der Zusammensetzung in einer wirksamen Menge hinzugefügt wird, die ausreicht, um α-Laktalbumin in einem der Bereiche bereitzustellen, die in menschlicher Muttermilch gefunden werden.

4. Synthetische Nährstoffzusammensetzung für einen Säugling oder ein Kind, umfassend einen α-Laktalbumin-angereicherten Molkenproteinextrakt nach Anspruch 1, wobei die synthetische Nährstoffzusammensetzung den WPE in einer Konzentration in einem Bereich von 0,8 bis 8 g/L umfasst und wobei die Cholesterinkonzentration in der Zusammensetzung mindestens 90 mg/L beträgt.

5. Synthetische Nährstoffzusammensetzung für einen Säugling oder ein Kind nach Anspruch 4, wobei die Zusammensetzung eine Säuglingsnahrung, umfassend 4 bis 5 g/L WPE, ist und wobei die Zusammensetzung vorzugsweise für einen Säugling in einem Alter von 6 bis 12 Monaten formuliert ist.

6. Synthetische Nährstoffzusammensetzung für einen Säugling oder ein Kind nach Anspruch 4, wobei die Zusammensetzung eine Säuglingsnahrung, umfassend 4 bis 5 g/L WPE, ist und wobei die Zusammensetzung vorzugsweise für ein Kind in dem Alter von 13 bis 36 Monaten formuliert ist.

7. Synthetische Nährstoffzusammensetzung für einen Säugling oder ein Kind nach Anspruch 4, wobei die Zusammensetzung eine Säuglingsnahrung, umfassend 0,5 bis 1,5 g/L WPE, ist und wobei die Zusammensetzung vorzugsweise für ein Kind in dem Alter von 3 bis 8 Jahren formuliert ist.

8. Synthetische Nährstoffzusammensetzung für einen Säugling oder ein Kind nach einem der Ansprüche 4 bis 7, wobei die synthetische Nährstoffzusammensetzung für einen Säugling oder ein Kind eine Zusammensetzung für einen Verzehr durch Säuglinge entweder allein oder in Kombination mit menschlicher Muttermilch ist und vorzugsweise eine Säuglingsnahrung oder ein Anreicherungsmittel für menschliche Muttermilch ist.

## Revendications

1. Utilisation d'un extrait de protéine de lactosérum (WPE) enrichi en α-lactalbumine en tant que source de cholestérol dans une composition nutritionnelle synthétique pour un nourrisson ou un enfant, dans laquelle le WPE enrichi en α-lactalbumine est obtenu par un procédé comprenant :
a. l'acidification d'un produit de protéine de lactosérum à pH 4 ou inférieur
b. la formation d'un produit de protéine de lactosérum à faible teneur en calcium en concentrant les protéines dans la protéine de lactosérum acidifiée jusqu'à ce que le rapport de calcium à protéine soit inférieur à environ 0,001 et,
c. la précipitation d'α-lactalbumine à partir du produit de protéine de lactosérum à faible teneur en calcium, dans laquelle ladite étape de précipitation comporte les sous-étapes consistant à :
(I) diluer le produit de protéine de lactosérum à faible teneur en calcium,
(II) ajuster le pH du produit de protéine de lactosérum à faible teneur en calcium dilué à entre 4 et 5 pour former un précipité et des protéines solubles, et
(III) séparer les protéines précipitées des protéines solubles ;
dans laquelle le WPE est ajouté à ladite composition en une quantité efficace suffisante pour assurer que ladite composition nutritionnelle synthétique ait une concentration finale de cholestérol présente dans le lait maternel humain.

2. Utilisation selon la revendication 1, dans laquelle la teneur en cholestérol dans une composition nutritionnelle synthétique va de 90 à 150 mg/L.

3. Utilisation selon les revendications 1 ou 2, dans laquelle le WPE est également utilisé pour optimiser la concentration d'α-lactalbumine dans ladite composition nutritionnelle synthétique pour un nourrisson ou un enfant, et dans laquelle ledit WPE est ajouté à ladite composition en une quantité efficace suffisante pour fournir de l'α-lactalbumine dans l'une des plages présentes dans le lait maternel humain.

4. Composition nutritionnelle synthétique pour un nourrisson ou un enfant comprenant un extrait de protéine de lactosérum enrichi en α-lactalbumine selon la revendication 1, dans laquelle la composition nutritionnelle synthétique comprend le WPE en une concentration dans une plage de 0,8 à 8 g/L et dans laquelle la concentration en cholestérol dans ladite composition est d'au moins 90 mg/L.

5. Composition nutritionnelle synthétique pour un nourrisson ou un enfant selon la revendication 4 dans laquelle ladite composition est une préparation pour nourrissons comprenant 4 à 5 g/L de WPE et dans laquelle ladite composition est de préférence formulée pour un nourrisson âgé de 6 à 12 mois.

6. Composition nutritionnelle synthétique pour un nourrisson ou un enfant selon la revendication 4 dans laquelle ladite composition est une préparation pour nourrissons comprenant 4 à 5 g/L de WPE et dans laquelle ladite composition est de préférence formulée pour un enfant âgé de 13 à 36 mois.

7. Composition nutritionnelle synthétique pour un nourrisson ou un enfant selon la revendication 4, dans laquelle ladite composition est une préparation pour nourrissons comprenant 0,5 à 1,5 g/L de WPE et dans laquelle ladite composition est de préférence formulée pour un enfant âgé de 3 à 8 ans.

8. Composition nutritionnelle synthétique pour un nourrisson ou un enfant selon l'une quelconque des revendications 4 à 7 dans laquelle la composition nutritionnelle synthétique pour un nourrisson ou un enfant est une composition pour la consommation par des nourrissons seule ou en combinaison avec du lait maternel humain et est de préférence une préparation pour nourrissons ou un fortifiant pour le lait maternel humain.
